# EUROPEAN PATENT APPLICATION

(11) **EP 3 621 084 A1**
(43) Date of publication of application: **11.03.2020**
(21) Application number: 18461606.8
(22) Date of filing: 10.09.2018
(51) Int. Cl.: G16H 20/30, G16H 20/70, A63B 71/06, G06F 3/01, A63F 13/212, A63F 13/21, G09B 19/00

(54) **A SYSTEM AND A METHOD FOR GENERATING A VIRTUAL REALITY ENVIRONMENT FOR EXERCISES VIA A WEARABLE DISPLAY**

(71) Applicant: Przedsiebiorstwo Produkcyjno Uslugowe "Stolgraf" Pasternak, Rodziewicz Spolka Jawna, 58-141 Stanowice (PL)
(72) Inventor: Mandzyn, Tomasz, 58-100 Swidnica (PL); Pasternak-Wyrwas, Dorota, 58-100 Swidnica (PL); Rodziewicz, Grzegorz, 58-100 Swidnica (PL); Pasternak, Pawel, 55-080 Krzeptow (PL)
(74) Representative: Kancelaria Eupatent.pl Sp. z.o.o

(57) **Abstract**

A method for generating a virtual reality environment for exercises to a user via a wearable display, the method being characterized in that it comprises the steps of: selecting (201) a mode of operation of the exercise; displaying (203) a virtual reality environment according to the selected mode of operation, on the wearable display; while the user performs the required steps of the exercise in the virtual reality environment, according to the selected mode of operation, recording (204) parametrized results of the exercise, wherein the parameters are measured as at least one of: time, distance, speed, accuracy; obtaining (205) user's feedback regarding the exercise session; analyzing (206) the recorded results of the exercise; and based on the analysis result and the user's feedback, providing (207) a suggestion for a next exercise.

## Description

### TECHNICAL FIELD

The present disclosure relates to a system and a method for generating a virtual reality environment for exercises via a wearable display. The presented method and system may be used for exercises useful for rehabilitation, especially in case of people recovering from stroke or brain injury. The brain injury may be a traumatic brain injury or other conditions that impact brain function.

### BACKGROUND

It has been found that low efficiency of physiotherapy of patients after stroke is conditioned by the presence of depression symptoms, low acceptance of the illness as well as lack of self-efficacy in the rehabilitation process.

Thus, it was found that it would be beneficial to put the patient in a state of relaxation that allows elimination of barriers against taking rehabilitation, because rehabilitation during the first three months after brain injury or stroke is critical in order to regain as much full health condition as possible. It was found that Erickson physiotherapy, which utilizes clinical hypnosis, also helps to improve the psychical state of a patient.

Prior art defines so-called 3D human-machine interfaces for multimedia and computer systems. These interfaces use a dedicated 3D positioning sensor (e.g. a depth sensor or a depth camera) which is configured to capture three dimensional representations of an observed scene comprising the user who inputs data into the system by movement (typically of fingers, hands, legs).

In typical solutions, the user interacts with a two-dimensional display, but may also interact with other controlled items or equipment reacting to special gestures the respective equipment is able to detect, process and interpret (for example a gesture for switching an equipment on/off).

Further, there are known so-called virtual reality (VR) systems. Current VR technology typically uses VR headsets to generate realistic images, sounds and/or other sensations that simulate a user's physical presence in a virtual environment. A person using VR equipment is able to navigate in the artificial world and interact with virtual items. VR headsets typically consist of a head-mounted display with a small screen positioned in front of the eyes. Some VR systems include transmission of vibrations and other sensations to the user through additional devices - such systems are known as haptic systems. This tactile response is generally known as force feedback and is commonly used in medical, video gaming and military training applications.

There exists a niche area where rehabilitation systems could benefit from application of virtual reality. Accordingly, there exists a need for methods, systems for providing rehabilitation using a wearable display.

It would be advantageous to allow a patient to do rehabilitation process without a need of a qualified assisting person. Preferably, such system could also use a pointing device and or other devices required for certain exercises (e.g. a ball).

Further, there is a need to provide a system to reduce symptoms of depression as well as strengthening the patient in the acceptance of the illness and self-efficacy in the process of physical and mental improvement.

### SUMMARY

There is presented herein a method for generating a virtual reality environment for exercises to a user via a wearable display, the method comprising the steps of: selecting a mode of operation of the exercise; displaying a virtual reality environment according to the selected mode of operation, on the wearable display; while the user performs the required steps of the exercise in the virtual reality environment, according to the selected mode of operation, recording parametrized results of the exercise, wherein the parameters are measured as at least one of: time, distance, speed, accuracy; obtaining user's feedback regarding the exercise session; analyzing the recorded results of the exercise; and based on the analysis result and the user's feedback, providing a suggestion for a next exercise.

The modes of operation can be selected from a group comprising: user's injury type selection, difficulty selection as well as test or exercise selection.

There is also presented a virtual reality environment for exercises using a wearable display, the system comprising: a data bus communicatively coupled to a memory wherein other components of the system are communicatively coupled to the system bus so that they may be managed by a controller; wherein the memory is configured to store computer program executed by the controller in order to execute steps of the method as presented herein; a communication module configured to exchange computer data with external interfaces or machines; a virtual reality module configured to generative a virtual, three-dimensional environment and appropriate graphical scenes according to the configuration of the system wherein such generated scenes are then output to the wearable display; and at least one input device configured to receive input from the user.

The input device may have labels or markers thereon that are detectable in 3D space.

The system may further comprise a 3D position sensor.

The system may further comprise a printing module configured to print recorded results of the exercise.

The system may further comprise a video signal output module configured to output a signal for an external display.

### BRIEF DESCRIPTION OF THE DRAWINGS

These and other objects of the disclosure presented herein, are accomplished by providing a system and method for generating the virtual reality environment for exercises using wearable display. Further details and features of the present disclosure, its nature and various advantages will become more apparent from the following detailed description of the preferred embodiments shown in a drawing, in which:
Fig. 1 presents a block diagram of the system according to the present disclosure;
Fig. 2 presents a diagram of the method according to the present disclosure;
Fig. 3 presents an embodiment of the system according to the present disclosure;
Fig. 4 presents a side view of the system in different compact and extended forms;
Fig. 5 shows a transportation container for the system according to the present disclosure;
Fig. 6 presents air flow within the casing of the main unit according to the present disclosure;
Fig. 7 shows an exploded view presenting components of an example of the main unit;
Fig. 8 illustrates drawings of computer screen shots of exemplary exercises that may be facilitated by the system according to the present disclosure; and
Figs 9 and 10 shown other drawings of computer screen shots of exemplary exercises that may be facilitated by the system according to the present invention.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

### DETAILED DESCRIPTION

The system according to the present disclosure is a plug and play system comprising a combination of hardware and software that is configured to allow performing an exercise in virtual reality by any person at any location. At the same time the system may be easily operated by any person and does not require any special skills from the operator. For example, one human operator may assist in setting up and controlling a plurality of exercise stations at the same time. Therefore the system is suitable for exercises for groups of persons.

Further, the system allows for constant monitoring of the user as well as progress the user is making. The results of monitoring may be stored and made available for further analysis.

The present system is meant as a uniform and complete solution ready for operation after it has been connected to a power source (plug&play). It does not require any setup or calibration while being easy to transport and operate by persons without any special training to this end.

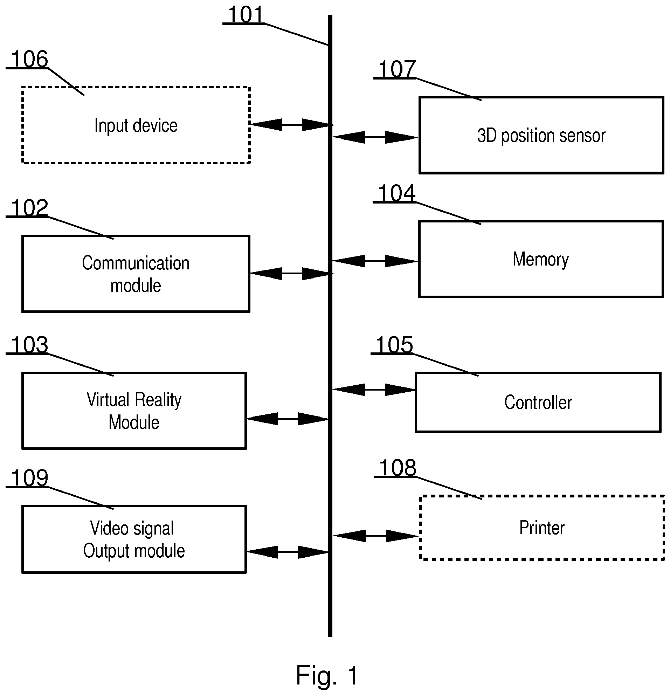

Fig. 1 presents a diagram of the system according to the present disclosure. The system is configured to facilitate interaction with a virtual reality environment and for example drawing and/or selection of objects in a suitable virtual reality environment especially adapted for exercises.

The system may be realized using dedicated components or custom made FPGA (Field-programmable gate array) or ASIC (Application-Specific Integrated Circuit) circuits. The system comprises a data bus 101 communicatively coupled to a memory 104. Additionally, other components of the system are communicatively coupled to the system bus 101 so that they may be managed by a controller 105.

The memory 104 may store computer program or programs executed by the controller 105 in order to execute steps of the method according to the present disclosure. Further, the memory 104 may store any temporary and/or final results of tests, evaluations or exercises.

A communication module 102 may comprise a wire (Ethernet cable) or wireless communication (e.g. Wi-Fi, 3G, LTE) used for exchanging computer data with external interfaces or machines. Such communication module 102 may also facilitate remote access, which may be used for support/help purposes and/or configuration/service purposes.

A virtual reality module 103 is responsible for generating a virtual, three-dimensional world and appropriate graphical scenes according to the configuration of the system. Such generated scenes are then output to suitable virtual reality goggles (a wearable display in general).

At least one input device 106 may be provided to a user so that the user may identify, select, or manipulate objects presented to the user using the virtual reality goggles. The input devices 106 may have labels/markers (either passive or active as known in the art) on them that make then more easily detectable in 3D space. In some cases, a specially adapted detection system may be employed for certain types of input devices.

In order to detect gestures made by the input devices 106, the present invention may utilize a 3D position sensor 107 such as a depth sensor, a depth camera or a triangular positioner. Such 3D position sensors are often called with different names such as ranging camera, flash lidar, time-of-flight (ToF) camera or RGB-D camera.

The underlying sensing mechanisms are also equally varied: range-gated ToF, RF-modulated ToF, pulsed-light ToF, and projected-light stereo.

Nevertheless, since the system according to the present disclosure is envisaged for everyday use and everyday devices, cost effective systems of input devices and 3D position sensors are preferred, such as those utilized by XTion Pro offered by ASUS or DS311 offered by SoftKinetic or Vive offered by HTC. It is typically sufficient for such a system to cover an area of a radius of few meters.

Optionally, more than one 3D sensor 107 is used, which may or may not be positioned at more than one angle of with respect to the user. Such arrangement may provide improved detection results.

Optionally, the present system may comprise or be connected to a printing module 108 or a printer. This may be useful for a user who may have his test result, evaluation result or exercise result printed.

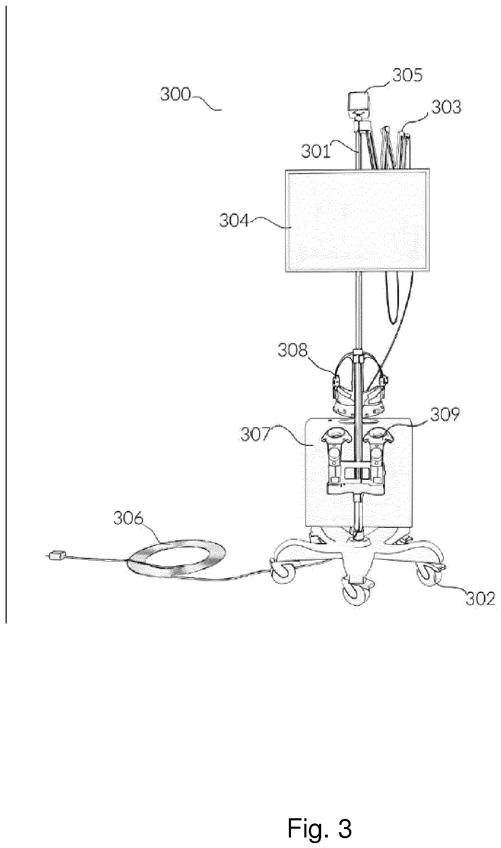

The system may further comprise a video signal output module 109 configured to output a signal for a suitable display as will be presented with reference to Fig. 3. Typically, such signal will comprise graphical user interface used for communication with a user and/or a service personnel.

The present system is meant as a uniform and complete solution ready for operation after it has been connected to a power source (plug&play). It does not require any setup or calibration while being easy to transport and operate by persons without any special training to this end.

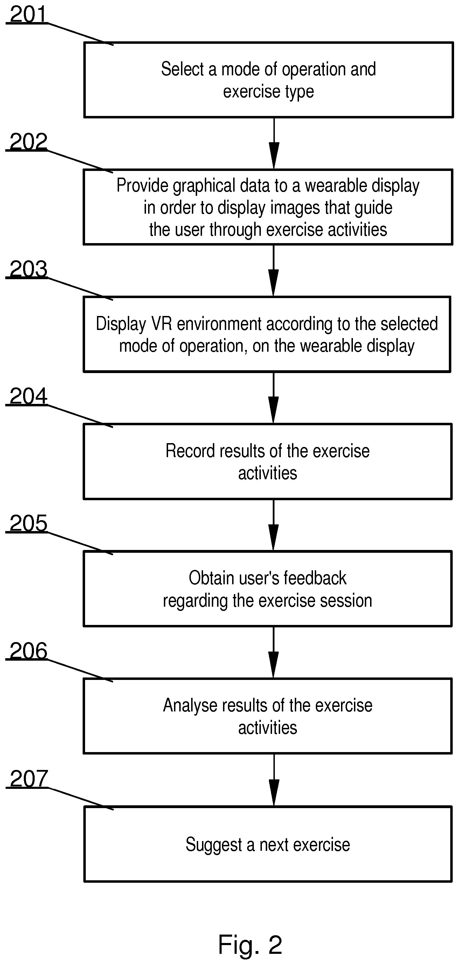

Fig. 2 presents a diagram of the method according to the present disclosure. The method starts at step 201 from selecting a mode of operation by a user or the exercise supervisor. The modes of operation are for example injury type selection, difficulty selection as well as test or exercise selection.

At step 202, there is provided graphical data to a wearable display in order to display images that guide the user through the exercise activities, i.e. a tutorial on how to perform the exercise.

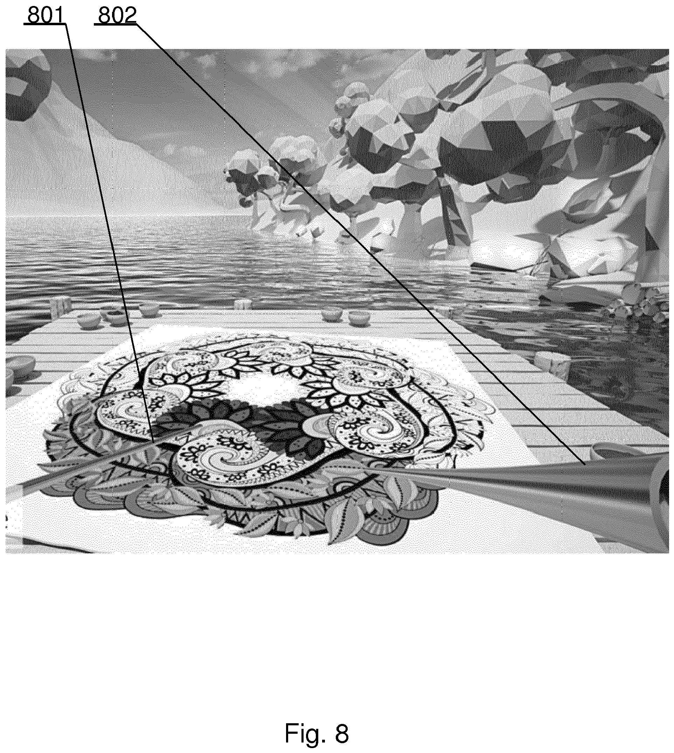

Next, at step 203 the system displays virtual reality environment according to the selected mode of operation, on the wearable display, for example the system displays a mandala drawing application as presented in Figs. 8 - 10.

While the user performs the required steps of the exercise, according to the selected mode of operation, the system records parametrized results 204 of the exercise activities. The parameters may be measured as time, distance, speed, percentage accuracy etc.

In particular, the following data may be collected during the exercise:
- the speed of movement: i.e. how quickly the user navigates through the exercise, e.g. to move the pointer from one position to another;
- the level of hand trembling: i.e. how stable is the position of the pointer navigated by the user;
- reaction time: i.e. how quickly the user starts to respond to instructions of the exercise;
- exercise completion time: i.e. how quickly the user completed the whole exercise or its fragments;
- the distance of subsequent moves: i.e. how distant was the location of the next activity compared to the previous activity;
- action accuracy: i.e. how precisely the user pointed to a particular location.

The data may be collected separately for different input arrangements, i.e. when the user navigates the input interface with the left hand or with the right hand.

Subsequently, at step 205, the system obtains user's feedback regarding the exercise session. When a session is complete the user may input an evaluation of the session comprising marks for, for example, difficulty setting, quality of the exercise process, user's mood, user's subjective performance evaluation etc. Such feedback may be input as a speech signal or input by a person helping the user.

Once these information is submitted, at step 206 there are analyzed results of the exercise activities, which are then compared to predefined ranges of results so that a next suggested exercise is suggested for the user in step 207. For example, if the user feedback was negative, another type of exercise may be suggested for the user. If the user feedback was positive and the results (e.g. the speed or accuracy) of the exercise were low, the next suggested exercise may be of the same type but lower difficulty level (e.g. to color a mandala image with lower number of elements). If the user feedback was positive and the results were high, the next suggested exercise may be of the same type but higher difficulty level (e.g. to color a mandala image with higher number of elements). If the user feedback was negative and the results were high, the next suggested exercise may be of a different type but with a higher difficulty level.

The respective exercises may train at least one of the following abilities of a user: coordination, vision, language, or cognition.

Once the user has selected the mode of operation, the system controls the virtual reality module 103 to provide graphical data to a wearable display in order to display images that guide the user through exercise activities corresponding to the selected mode of operation.

The system provides feedback to the user/assistant. The feedback may include timing, results data, video, or other indication of the outcome of the user's exercise session.

Fig. 3 presents an embodiment of the system 300 according to the present disclosure. The system is complete in a sense that it only requires plugging it to a power source 306.

The key elements of the system are a stand 301 having attached wheels 302 in order to facilitate its easy movement and transportation. The stand 301 is preferably configurable to allow different height levels of its monitor 304 (which may be a touch screen) and extension arm 304.

A sensor 305, such as a depth camera or a triangular positioner, may be mounted at one end of the stand 301 or alternatively on a suitable extension arm.

A main unit 307 is preferably located near a base of the stand as it may be the heaviest component and placing it low results in better stability of the whole stand 301.

The stand 301 may further comprise special adapters of elements suitable to keep or otherwise store a set of virtual reality goggles 308 as well as any required hardware controllers or headphones, which may further have their own docking stations

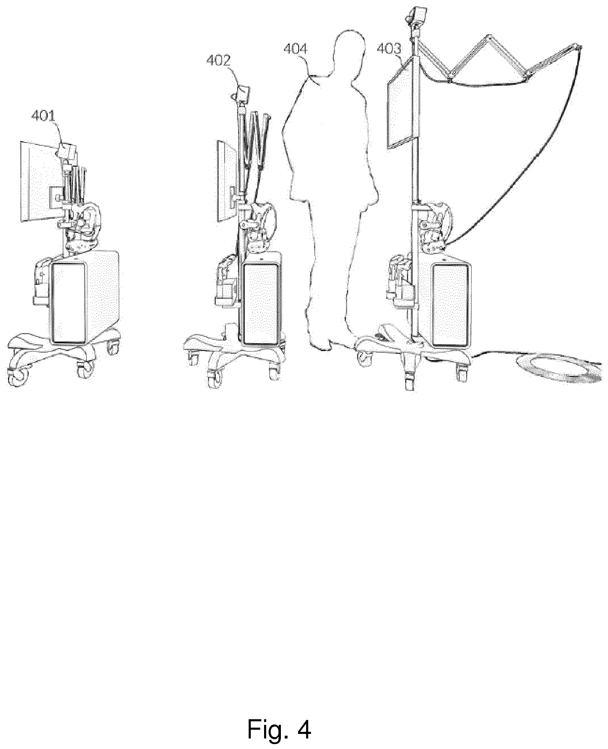

Fig. 4 presents a side view of the system in a minimized form 401 wherein the stand 301 is reduced and the extensible arm 303 is folded. The stand 301 and the extensible arm 303 may be suitably extended 402 in order to match the height 403 of the exercising user 404.

Additionally, the extensible arm 303 may rotate about the stand in case any wires are needed between the main unit 307 and the virtual reality goggles 308.

In some embodiments of the disclosure, the user may also sit (from example in a wheelchair) in front of the stand 301 comprising the system according to the present disclosure.

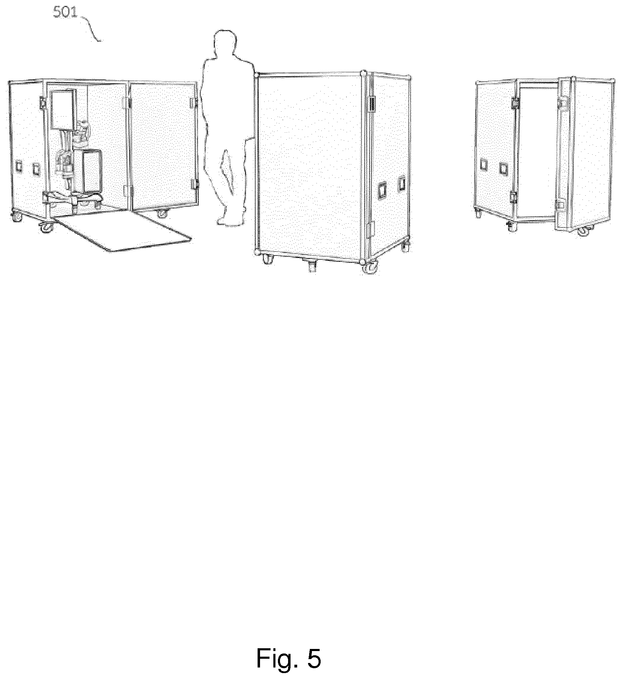

Fig. 5 shows a transportation container for the system 300. Prior to being placed in said container 501 the system is reduced to its most compact form 401. The transportation container 501 may also be equipped with a suitable lock as well as wheels facilitating its easy movement. The transportation container 501 also serves a function of protecting the system from damage when it is not in use for exercising.

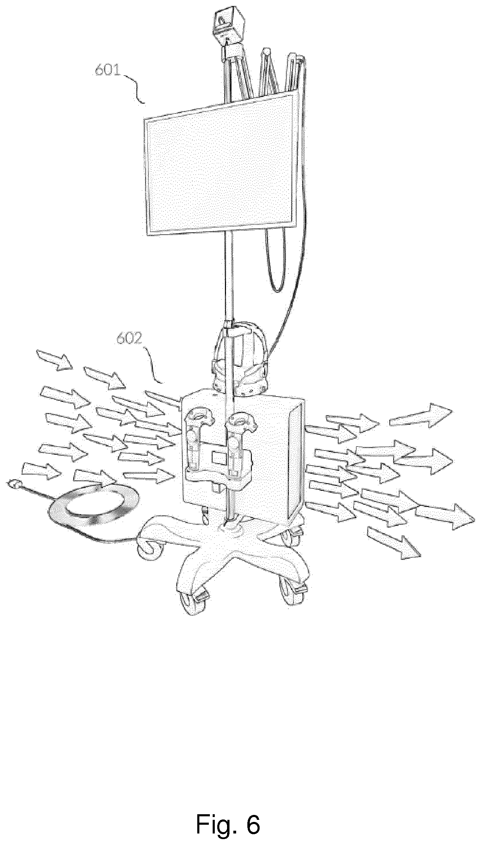

Fig. 6 presents air flow 601 within the casing of the main unit 307 according to the present disclosure. As may be seen, cold air if pulled inside the casing and hot air is pushed out of the casing. One direction of the airflow is maintained while the casing has its airflow are 602 as large as possible.

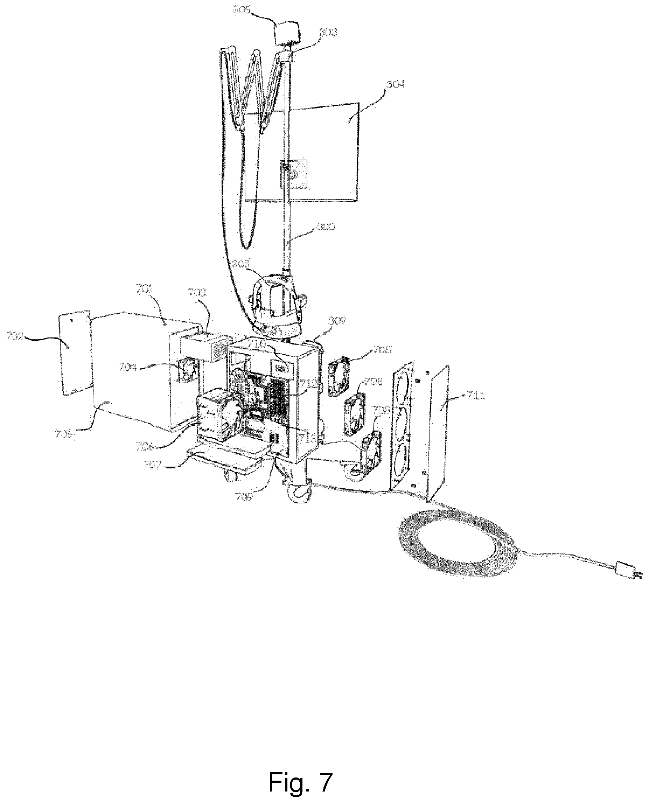

Fig. 7 shows an exploded view presenting components of an example of the main unit 307 of the system according to the present disclosure. This implementation comprises specific embodiments of most modules presented with reference to Fig. 1.

A chassis 705 holds all key components and is preferably made of a material well conducting heat, such as aluminum. A power on/off button 701 is preferably positioned on top of the chassis for easy access, nevertheless other locations thereof are possible.

A power supply unit 703 is mounted within the chassis and comprises a power cord for connecting the power from a wall socket. The power supply unit 703 is also configured to provide all levels of voltage required by the internal components of the system, which may operate on different voltages.

A plurality of air cooling modules 704, 706, 708 such as fans may be provided to cool respective components and provide sufficient airflow within the chassis 705.

A suitable graphic card 707 for example in SLI (Scalable Link Interface) configuration is preferred since generation of virtual reality environments may be heavy on graphics data processing. Other GPU configurations are naturally possible as long as the GPU capability is sufficient to execute all required tasks.

A mainboard 709 may be configured to organize all components of the system and provide suitable interfaces for the main processor 713, RAM memory 712 as well as non-volatile memory 710 such as an SSD drive.

At least one and in most cases two covers 702, 711, preferably of hardened glass, may be provided so that the internal components of the system are visible from the outside of the chassis 705.

Fig. 8 illustrates drawings of computer screen shots of exemplary exercise activities that may be facilitated by the system according to the present disclosure. Objects 801, 802 may be graphical representations of user's controllers operated by user's hands.

In this case a user draws or fills blank spaces with color. A preferred image is a mandala picture, which is typically a complex image with a lot of small elements that may be colored in order to form a pattern. The user may be instructed to navigate the objects 801, 802 such as to touch specific areas of the mandala. The difficulty of the exercise may be adjusted by providing a mandala image with a lower or a higher number of elements, or by providing instructions to the user to perform the actions slower or quicker. The results of the exercise may be measured for example by determining:
- how quickly the user moves the objects 801, 802;
- how accurately the user points to specific elements of the image;
- how stable is the movement, what is the level of trembling of the hand;
- how the accuracy changes over the time (whether the user gets tired during the exercise).

Moreover, the results may be compared for the objects 801, 802 navigated by the left hand and the right hand.

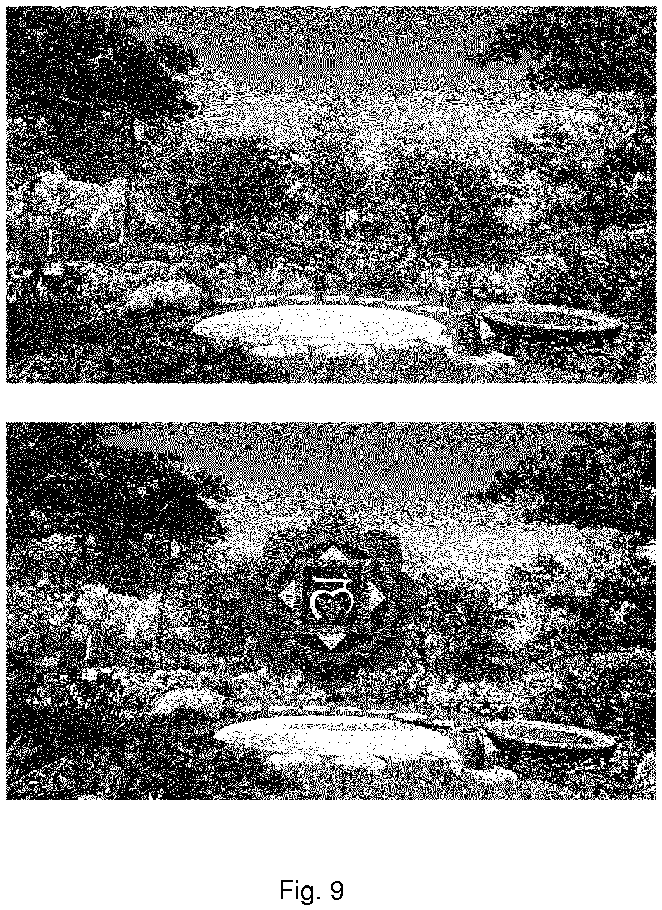

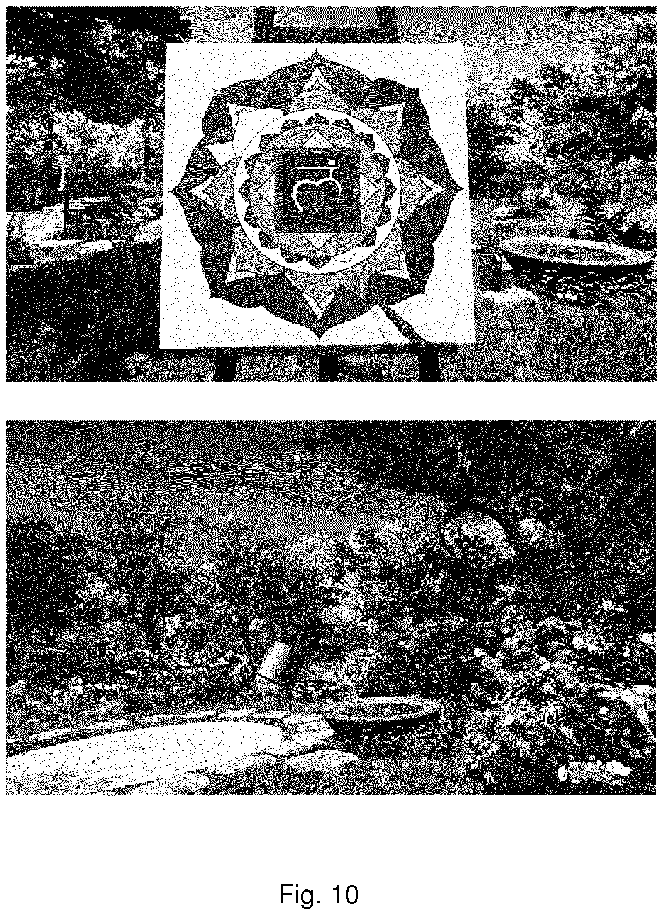

Figs 9 and 10 shown other drawings of computer screen shots of exemplary exercise activities that may be facilitated by the system according to the present invention.

The mandala image may be displayed within a virtual reality environment, such as a garden, via which the user may navigate to have a more realistic experience. The environment may change over time, for example the garden may flourish as the user performs well the exercise.

To this end different options may be provided to a user. The first option is just revealing colors of mandala items/tiles, wherein such times are selected by the system and a user only clicks on the pre-selected tile.

The second option is mandala coloring wherein a user selects colors and then selects a tile for which the color is to be applied. The most difficult option is drawing a mandala picture from scratch or completing a partially drawn mandala picture.

Another option may be remembering colors of a mandala i.e. a colored mandala is shown to a user, then some or all colors are removed and the user is asked to color the mandala such that it looks the same as the initially shown mandala.

A session with a user may comprise more than one type of the aforementioned exercises related to mandalas.

Each option may be provided with different difficulty levels such as time constraints, number of tiles, amount of instructions given via the headphones or diversity of the tiles.

The use of such mandala games leads to psycho-physical relaxation, improved mood and concentration, improved visuospatial function as well as increased brain plasticity.

The present disclosure provides a system that facilitates exercising using wearable display and a special virtual reality system tailored for users to improve their skills. Therefore, the disclosure provides a useful, concrete and tangible result.

The present system is meant as a uniform and complete solution ready for operation after it has been connected to a power source (plug&play). It does not require any setup or calibration while being easy to transport and operate by persons without any special training to this end.

Further, the present disclosure defines a machine suitable to achieve the aforementioned effects. Therefore, the machine or transformation test is fulfilled and that the idea is not abstract.

At least parts of the methods according to the disclosure may be computer implemented. Accordingly, the present disclosure may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present disclosure may take the form of a computer program product embodied in any tangible medium of expression having computer usable program code embodied in the medium.

It can be easily recognized, by one skilled in the art, that the aforementioned method for providing exercises using wearable display may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the disclosure presented herein has been depicted, described, and has been defined with reference to particular preferred embodiments, such references and examples of implementation in the foregoing specification do not imply any limitation on the disclosure. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A method for generating a virtual reality environment for exercises to a user via a wearable display, the method being **characterized in that** it comprises the steps of:
- selecting (201) a mode of operation of the exercise;
- displaying (203) a virtual reality environment according to the selected mode of operation, on the wearable display;
- while the user performs the required steps of the exercise in the virtual reality environment, according to the selected mode of operation, recording (204) parametrized results of the exercise, wherein the parameters are measured as at least one of: time, distance, speed, accuracy;
- obtaining (205) user's feedback regarding the exercise session;
- analyzing (206) the recorded results of the exercise; and
- based on the analysis result and the user's feedback, providing (207) a suggestion for a next exercise.

2. The method according to claim 1 wherein the modes of operation are selected from a group comprising: user's injury type selection, difficulty selection as well as test or exercise selection.

3. A system for generating a virtual reality environment for exercises using a wearable display, the system being **characterized in that** it comprises:
- a data bus (101) communicatively coupled to a memory (104) wherein other components of the system are communicatively coupled to the system bus (101) so that they may be managed by a controller (105);
- wherein the memory (104) is configured to store computer program executed by the controller (105) in order to execute steps of the method according to any of previous claims;
- a communication module (102) configured to exchange computer data with external interfaces or machines;
- a virtual reality module (103) configured to generative a virtual, three-dimensional environment and appropriate graphical scenes according to the configuration of the system wherein such generated scenes are then output to the wearable display; and
- at least one input device (106) configured to receive input from the user.

4. The system according to claim 3 wherein the input device (106) has labels or markers thereon that are detectable in 3D space.

5. The system according to any of claims 3-4 wherein the system further comprises a 3D position sensor (107).

6. The system according to any of claims 3-5 wherein the system further comprises a printing module (108) configured to print recorded results of the exercise.

7. The system according to any of claims 3-6 wherein the system further comprises a video signal output module (109) configured to output a signal for an external display.
